(19) Europäisches Patentamt
European Patent Office
Office européen des brevets

(11) Veröffentlichungsnummer: **0 017 614**
**B1**

# (12) EUROPÄISCHE PATENTSCHRIFT

(45) Veröffentlichungstag der Patentschrift: **25.08.82**

(51) Int. Cl.³: **C 07 C 149/18**

(21) Anmeldenummer: **80810075.4**

(22) Anmeldetag: **03.03.80**

(54) **Verfahren zur Herstellung von Estern von Mercapto-alkanolen mit Hydroxyphenylcarbonsäuren.**

(30) Priorität: **09.03.79 CH 2280/79**
**03.05.79 CH 4141/79**

(43) Veröffentlichungstag der Anmeldung:
**15.10.80 Patentblatt 80/21**

(45) Bekanntmachung des Hinweises auf die Patenterteilung:
**25.08.82 Patentblatt 82/34**

(84) Benannte Vertragsstaaten:
**BE CH DE FR GB IT NL**

(56) Entgegenhaltungen:
**FR-A-1 478 041**
**FR-A-2 332 806**
**GB-A-1 061 780**
**US-A-3 109 021**
**US-A-3 960 741**

(73) Patentinhaber: **CIBA-GEIGY AG, Patentabteilung Postfach, CH-4002 Basel (CH)**

(72) Erfinder: **Rosenberger, Siegfried, Im Gehracker 11, CH-4125 Riehen (CH)**

EP 0 017 614 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Estern von Mercaptoalkanolen mit Hydroxyphenylcarbonsäuren

Die Erfindung betrifft ein Verfahren zur Herstellung von Estern von Mercaptoalkanolen mit Hydroxyphenylcarbonsäuren in einer Stufe mit annähernd quantitativer Ausbeute.

Die FR-PS 2 018 870 beschreibt die Herstellung von 3,5-Di-tert.-butyl-4-hydroxyphenylpropionsäure-2-mercaptoäthylester. Sie sind gemäß FR-PS 2 018 870 als Zwischenprodukte für die Herstellung von Stabilisatoren für polymere Materialien geeignet. In einem 2-Stufen-Verfahren wird zuerst der 3,5-Dialkyl-4-hydroxyphenylcarbonsäuremethylester verseift und anschließend mit dem Mercaptoalkohol verestert. Die FR-PS 2 018 870 beschreibt die Veresterungsstufe in Gegenwart eines sauren Katalysators.

Um die Nachteile eines 2-Stufen-Verfahrens zu eliminieren, hat man nach Wegen für eine direkte Umesterung gesucht. Es hat sich gezeigt, daß die direkte Umesterung mit sauren Katalysatoren nur sehr schlecht gelingt. Auch Versuche mit basischen Katalysatoren, wie $LiNH_2$ führen zu keinem befriedigenden Ergebnis.

Die bisherigen Erfahrungen lehrten, daß infolge der reaktiven freien Mercaptogruppe das gewünschte Endprodukt entweder in zu geringen Mengen erhalten wurde, oder so unrein, daß es mehrfach gereinigt werden mußte.

Verfahren zur Herstellung von Alkylmercaptoalkylestern oder von Diestern von Thiodialkanolen enthaltend 3,5-Dialkyl-4-hydroxyphenylalkanoylgruppen, durch Umesterung in Gegenwart geeigneter Katalysatoren sind jeweils in der US-PS 3 109 021, in der GB-PS 1 081 789 und in der FR-PS 1 479 641 beschrieben. In keinem der darin beschriebenen Verfahren tritt jedoch das durch das Verfahren der vorliegenden Anmeldung gelöste Problem zutage, nämlich die mit der reaktiven freien Mercaptogruppe zusammenhängenden Schwierigkeiten.

Es wurde nun ein unkompliziertes Verfahren zur Herstellung der Verbindung der Formel I gefunden, das überraschenderweise in einer Stufe durchgeführt werden kann und ausgezeichnete Ausbeuten liefert.

Gegenstand der vorliegenden Erfindung ist daher ein Verfahren zur Herstellung von Phenolen der Formel I

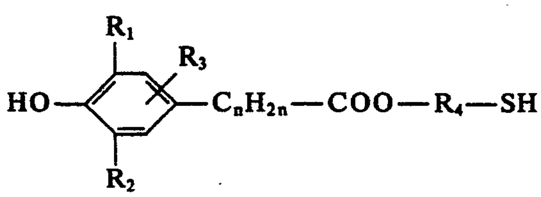 (I)

worin $R_1$ Alkyl mit 1 bis 18 C-Atomen, Cycloalkyl, Phenyl, Benzyl, α-Phenyläthyl, 2-Phenyl-isopropyl oder Chlor ist, $R_2$ Wasserstoff oder die Bedeutung von $R_1$ besitzt, $R_3$ Wasserstoff oder $C_1-C_{12}$-Alkyl, $R_4$ gegebenenfalls mit Phenyl oder Hydroxy substituiertes $C_2-C_{18}$-Alkylen und n eine ganze Zahl zwischen 0 und 4 bedeutet, dadurch gekennzeichnet, daß man eine Verbindung der Formel II

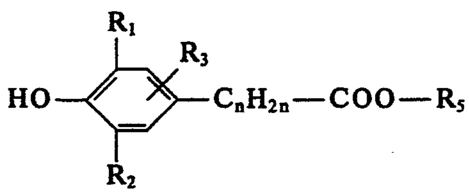 (II)

mit einem Mercaptoalkohol der Formel

$$HO-R_4-SH$$

worin $R_1$, $R_2$, $R_3$ und $R_4$ die genannte Bedeutung besitzen und $R_5$ Alkyl mit 1 bis 4 C-Atomen bedeutet, in Gegenwart eines Katalysators der Formel

$$(R_6O)_4-M$$

worin $R_6$ gleiche oder verschiedene Alkyl-Reste mit jeweils 1 bis 18 C-Atomen, Aryl oder Alkaryl bedeutet und M das Element Ti, Ge, Zr, Sn oder V ist, umestert.

Die Reaktanten können im Molverhältnis von 1 : 1 bis 1 : 3, bezogen auf den Ester der Formel II zum Mercaptoalkohol, eingesetzt werden. Bevorzugt ist das Molverhältnis 1 : 1 bis 1 : 2. Der Katalysator kann vorzugsweise in den Mengen von 0,01 bis 2,0 Mol-% verwendet werden. Besonders bevorzugt sind 0,1 bis 1,0 Mol-%.

Die Umsetzung kann bei 50 bis 200°C, bevorzugt bei 120 bis 180°C während 5 bis 25 Stunden erfolgen.

Das Verfahren kann sowohl in Gegenwart als auch in Abwesenheit eines durchgeführt werden. Verwendet man ein Lösungsmittel so kommen gegebenenfalls chlorierte aliphatische und aromatische Kohlenwasserstoffe sowie Äther in Frage, wie z. B. Ligroin, Benzol, Toluol, Chloroform, Chlorbenzol, Dichloräthan, Dioxan oder Diäthyläther.

Bevorzugt wird die Reaktion in Abwesenheit eines Lösungsmittels durchgeführt.

$R_1$ und $R_2$ können $C_1-C_{18}$-Alkyl bedeuten, wie z. B. Methyl, iso-Propyl, n-Butyl, sec.-Butyl, tert.-Butyl, tert.-Amyl, 2-Äthylhexyl, n-Octyl, tert.-Octyl, n-Dodecyl, 1,1,7,7-Tetramethyl-octyl, n-Octadecyl. $\alpha$-verzweigte Alkyl-Radikale mit 3 bis 8 C-Atomen sind bevorzugt, z. B. iso-Propyl, tert.-Butyl, tert.-Amyl und tert.-Octyl. Besonders bevorzugt ist tert.-Butyl, aber auch Methyl.

$R_1$ und $R_2$ können Cycloalkyl bedeuten, wie z. B. Cyclopropyl, Cyclopentyl, Cyclohexyl, Cycloheptyl, Cyclooctyl oder Cyclododecyl. Bevorzugt sind Cyclopentyl und Cyclohexyl.

Tert.-Octyl bedeutet jeweils 1,1,3,3-Tetramethyl-butyl.

$R_1$ und $R_2$ sind bevorzugt tert.-Butyl.

$R_3$ kann Wasserstoff oder $C_1-C_{12}$-Alkyl bedeuten. Das Alkyl-Radikal kann Methyl, Äthyl, iso-propyl, n-Butyl, tert.-Butyl, 2-Äthylhexyl, tert.-Octyl, n-Decyl, 1,1-Dimethyldecyl, vorzugsweise Methyl bedeuten.

Bevorzugt ist $R_3$ Wasserstoff.

$R_4$ kann $C_2-C_{18}$-Alkylen sein, z. B. 1,2-Äthylen, 1,2-Propylen, 1,3-Propylen, 1,2-Butylen, 1,4-Butylen, 1,6-Hexamethylen, 2,9-Decylen oder 1,16-Hexadecamethylen. Bevorzugt ist $R_4$ $C_2-C_4$-Alkylen, wie z. B. 1,2-Äthylen, 1,3-Propylen und 1,4-Butylen. Besonders bevorzugt ist 1,2-Äthylen.

n ist eine ganze Zahl zwischen 0 und 4, bevorzugt 1 bis 4. Besonders bevorzugt ist n 2 oder 4.

Bedeutet $R_6$ $C_1-C_{18}$-Alkyl, so kommen die gleichen Reste wie für $R_1$ beschrieben in Frage. Bevorzugt ist $R_6$ $C_1-C_4$-Alkyl, besonders bevorzugt n-Propyl oder n-Butyl.

Bedeutet $R_6$ Aryl so kann es z. B. Phenyl, $\alpha$-Naphthyl oder $\beta$-Naphthyl sein. Bevorzugt ist Phenyl.

Bedeutet $R_6$ Alkaryl, so kann es beispielsweise Tolyl, Xylyl, 2,6-Diäthylphenyl oder 4-tert.-Butylphenyl sein.

M ist vorzugsweise Ti.

Beispiele für die Verbindungen der Formel I sind:

2,3-Dimethyl-5-t-butyl-4-hydroxyphenyl(2,2-dimethyl)-propionsäure-2-mercaptoäthylester,
2,3-Dimethyl-5-t-butyl-4-hydroxyphenylpropionsäure-2-mercapto-2-phenyläthylester,
3-Methyl-5-t-butyl-4-hydroxyphenylpropionsäure-2-mercaptoäthylester,
3,5-Di-t-octyl-4-hydroxyphenylpropionsäure-2-mercaptopropylester,
3,5-Di-t-butyl-4-hydroxyphenylvaleriansäure-2-mercaptobutylester,
3,5-Di-t-butyl-4-hydroxyphenylpropionsäure-2-mercaptoäthylester,
3,5-Di-t-butyl-4-hydroxybenzoesäure-2-mercaptoäthylester.

Die Verbindungen der Formel II werden in bekannter Weise, wie in US 3 247 240 beschrieben, hergestellt.

Die Verbindungen der Formel I werden nach dem erfindungsgemäßen Einstufen-Verfahren in hoher Ausbeute und großer Reinheit erhalten.

Die nachfolgenden Beispiele erläutern die Erfindung.

## Beispiel 1

### 3,5-Di-t-butyl-4-hydroxyphenylpropionsäure-2-mercaptoäthylester

438 g 3,5-Di-t-butyl-4-hydroxyphenylpropionsäuremethylester werden im Reaktionsgefäß bei 155°C geschmolzen und mit 1,5 ml Tetra-n-butyl-ortho-titanat versetzt. Dann wird die Apparatur, welche mit einem auf 70°C erwärmten Rückflußkühler und anschließendem absteigenden Kühler und Kältefalle ausgerüstet ist, auf ca. 200 mm Hg evakuiert. Unter Rühren werden nun innerhalb von 4 Stunden 140 g 2-Mercaptoäthanol zugetropft und das Gemisch wird anschließend 5 Stunden weitergerührt. Dann werden nochmals 36 g 2-Mercaptoäthanol innerhalb 1 Stunde zugegeben und das Reaktionsgemisch wird noch weitere 7 Stunden bei 155°C und 300 mm Hg gerührt. Dabei destillieren etwa 55 ml Methanol ab. Im Gaschromatogramm ist jetzt praktisch kein Edukt mehr sichtbar.

Der Kolbeninhalt wird bei ca. 100°C mit 700 ml Toluol und 30 g Bleicherde Tonsil AC® (Fa. Süd-Chemie, München) versetzt, kurz aufgekocht, filtriert und das Toluol in Vakuum aus dem Filtrat entfernt.

Man erhält so das chromatographisch reine Produkt, welches beim Animpfen zu einer schwach gelblich gefärbten Kristallmasse erstarrt. Schmelzpunkt 50°C. Die Ausbeute beträgt 485 g, das sind 95% der Theorie, bezogen auf den eingesetzten Methylester.

## Beispiel 2

### 3-Methyl-5-t-butyl-4-hydroxyphenylpropionsäure-2-mercaptoäthylester

Man verfährt wie im Beispiel 1 mit dem Unterschied, daß man an Stelle von 3,5-Di-t-butyl-4-hydroxyphenylpropionsäuremethylester nun 3-Methyl-5-t-butyl-4-hydroxyphenylpropionsäuremethylester verwendet.

Der entstandene 3-Methyl-5-t-butyl-4-hydroxyphenylpropionsäure-2-mercaptoäthylester ist ein fast farbloses, viskoses Öl; es wurde mit gleich hoher Ausbeute und Qualität wie das Produkt im Beispiel 1, erhalten.

## Patentansprüche

1. Verfahren zur Herstellung von Verbindungen der Formel I

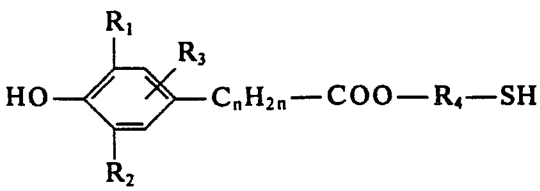

(I)

worin $R_1$ Alkyl mit 1 bis 18 C-Atomen, Cycloalkyl, Phenyl, Benzyl, $\alpha$-Phenyläthyl, 2-Phenyl-isopropyl oder Chlor ist, $R_2$ Wasserstoff oder die Bedeutung von $R_1$ besitzt, $R_3$ Wasserstoff oder $C_1-C_{12}$-Alkyl, $R_4$ gegebenenfalls mit Phenyl oder Hydroxy substituiertes $C_2-C_{18}$-Alkylen und n eine ganze Zahl zwischen 0 und 4 bedeutet, dadurch gekennzeichnet, daß man eine Verbindung der Formel II

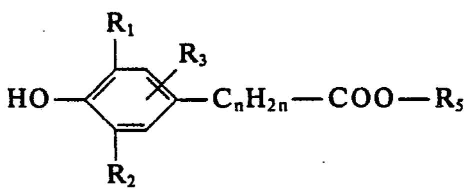

(II)

mit einem Mercaptoalkohol der Formel

$$HO-R_4-SH$$

worin $R_1$, $R_2$, $R_3$ und $R_4$ die in Anspruch 1 genannte Bedeutung besitzen und $R_5$ Alkyl mit 1 bis 4 C-Atomen bedeutet, in Gegenwart eines Katalysators der Formel

$$(R_6O)_4-M$$

worin $R_6$ gleiche oder verschiedene Alkyl-Reste mit jeweils 1 bis 18 C-Atomen, Aryl oder Alkaryl bedeutet und M das Element Ti, Ce, Zr, Sn oder V ist, umestert.

2. Verfahren nach Anspruch 1 zur Herstellung von Verbindungen der Formel I

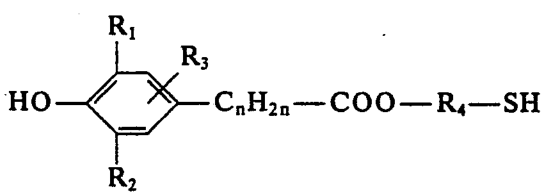

(I)

worin $R_1$ Alkyl mit 1 bis 18 C-Atomen, Cycloalkyl, $\alpha$-Phenyläthyl oder 2-Phenyl-isopropyl ist, $R_2$ Wasserstoff oder die Bedeutung von $R_1$ besitzt, $R_3$ Wasserstoff oder $C_1-C_{12}$-Alkyl, $R_4$ gegebenenfalls mit Phenyl substituiertes $C_2-C_{18}$-Alkylen und n eine ganze Zahl zwischen 0 und 4 b bedeutet, dadurch gekennzeichnet, daß man eine Verbindung der Formel II

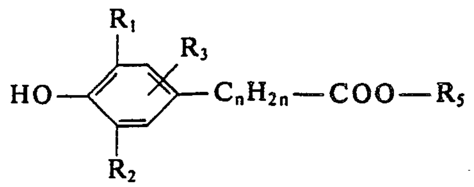

(II)

mit einem Mercaptoalkohol der Formel

$$HO-R_4-SH$$

worin $R_1$, $R_2$, $R_3$ und $R_4$ die vorstehend genannte Bedeutung besitzen und $R_5$ Alkyl mit 1 bis 4 C-Atomen bedeutet, in Gegenwart eines Katalysators der Formel

$$(R_6O)_4-M$$

worin $R_6$ gleiche oder verschiedene Alkyl-Reste mit jeweils 1 bis 18 C-Atomen bedeutet und M das Element Ti ist, umestert.

3. Verfahren nach Anspruch 1, dadurch gekennzeichnet, daß man als Katalysator Tetra-alkyl-ortho-titanat verwendet, worin das Alkyl gleich oder verschieden ist und jeweils 1 bis 4 C-Atome enthält.

4. Verfahren nach Anspruch 1, dadurch gekennzeichnet, daß man bei 50 bis 200° C umestert.

5. Verfahren nach Anspruch 1, dadurch gekennzeichnet, daß man bei 120 bis 180° C umestert.

6. Verfahren zur Herstellung von Verbindungen der Formel I gemäß Anspruch 1, worin $R_1$ und $R_2$ $C_1-C_8$-Alkylen, Cyclopentyl oder Cyclohexyl, $R_3$ Wasserstoff, $R_4$ $C_2-C_4$-Alkylen oder die Gruppe Phenyläthylen und n eine ganze Zahl zwischen 1 und 4 bedeutet.

7. Verfahren zur Herstellung von Verbindungen der Formel I gemäß Anspruch 1, worin $R_1$ und $R_2$ $\alpha$-verzweigtes Alkyl mit 3 bis 8 C-Atomen, $R_3$ Wasserstoff, $R_4$ Äthylen, 1,3-Propylen oder 1,4-Butylen und n die Zahl 2 oder 4 bedeutet.

8. Verfahren zur Herstellung von 3,5-Di-t-butyl-4-hydroxyphenylpropionsäure-2-mercaptoäthylester gemäß Anspruch 1.

## Claims

1. A process for the manufacture of a compound of the formula I

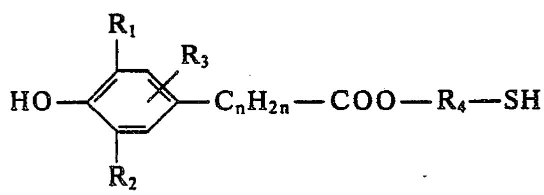

(I)

wherein $R_1$ is alkyl of 1 to 18 carbon atoms, cycloalkyl, phenyl, benzyl, $\alpha$-phenylethyl, 2-phenylisopropyl or chlorine, $R_2$ is hydrogen or has the same meaning as $R_1$, $R_3$ is hydrogen or alkyl of 1 to 12 carbon atoms, $R_4$ is alkylene of 2 to 18 carbon atoms which is unsubstituted or substituted by phenyl or hydroxyl, and n is an integer from 0 to 4, which process comprises transesterifying a compound of the formula II

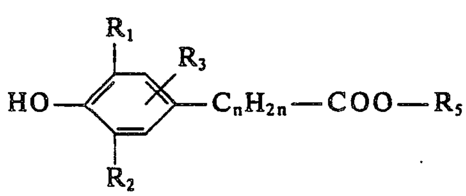

(II)

wherein $R_1$, $R_2$ and $R_3$ are as defined above and $R_5$ is alkyl of 1 to 4 carbon atoms, with a mercapto alcohol of the formula

$$HO-R_4-SH$$

wherein $R_4$ is as defined above, in the presence of a catalyst of the formula

$$(R_6O)_4\!-\!M$$

**wherein $R_6$ represents alkyl of 1 to 18 carbon atoms which can be the same or different, aryl or alkaryl, and M is an element selected from the group consisting of Ti, Ge, Zr, Sn or V.**

**2. A process according to claim 1 for the manufacture of a compound of the formula I**

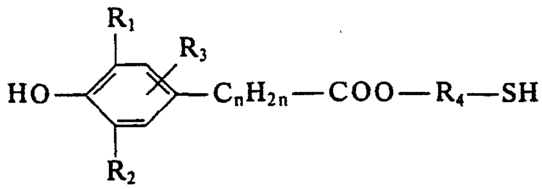

(I)

**wherein $R_1$ ist alkyl of 1 to 18 carbon atoms, cycloalkyl, $\alpha$-phenylethyl or 2-phenylisopropyl, $R_2$ is hydrogen or has the same meaning as $R_1$, $R_3$ is hydrogen or alkyl of 1 to 12 carbon atoms, $R_4$ is alkylene of 2 to 18 carbon atoms which is unsubstituted or substituted by phenyl, and n is an integer from 0 to 4, which process comprises transesterifying a compound of the formula II**

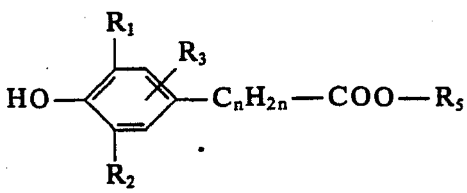

(II)

wherein $R_1$, $R_2$ and $R_3$ are as defined above and $R_5$ is alkyl of 1 to 4 carbon atoms, with a mercapto alcohol of the formula

$$HO\!-\!R_4\!-\!SH$$

wherein $R_4$ is as defined above, in the presence of a catalyst of the formula

$$(R_6O)_4\!-\!M$$

**wherein $R_6$ represents alkyl of 1 to 18 carbon atoms which can be the same or different, and M is Ti.**

**3. A process according to claim 1, wherein the catalyst is tetraalkyl-ortho-titanate, in which the alkyl moieties are the same or different and each contain 1 to 4 carbon atoms.**

**4. A process according to claim 1, wherein the transesterification is carried out in the temperature range from 50° to 200° C.**

**5. A process according to claim 1, wherein the transesterification is carried out in the temperature range from 120° to 180° C.**

**6. A process for the manufacture of a compound of the formula I according to claim 1, wherein $R_1$ and $R_2$ are $C_1$—$C_8$alkyl, cyclopentyl or cyclohexyl, $R_3$ is hydrogen, $R_4$ is $C_2$—$C_4$alkylene or the phenylethylene group and n is an integer from 1 to 4.**

**7. A process for the manufacture of a compound of the formula I according to claim 1, wherein $R_1$ and $R_2$ are $\alpha$-branched alkyl of 3 to 8 carbon atoms, $R_3$ is hydrogen, $R_4$ is ethylene, 1,3-propylene or 1,4-butylene, and n is 2 or 4.**

**8. A process for the manufacture of 2-mercaptoethyl 3,5-di-tert-butyl-4-hydroxyphenylpropionate.**

**Revendications**

**1. Procédé de préparation de composés répondant à la formule (I):**

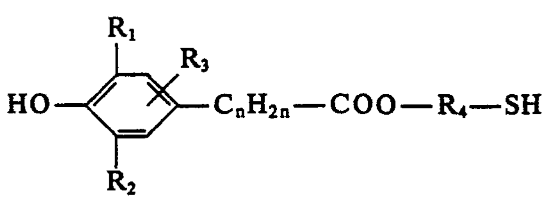

(I)

**dans laquelle $R_1$ représente un alkyle contenant de 1 à 18 atomes de carbone, un cycloalkyle, un phényle, un benzyle, un phényl-1 éthyle, un phényl-2 isopropyle ou le chlore, $R_2$ représente**

l'hydrogène ou un alkyle contenant de 1 à 12 atomes de carbone, $R_4$ représente un alkylène en $C_2-C_{18}$ éventuellement porteur d'un phényle ou d'un hydroxy, et n désigne un nombre entier de 0 à 4, procédé caractérisé en ce qu'on transestérifie un composé de formule (II)

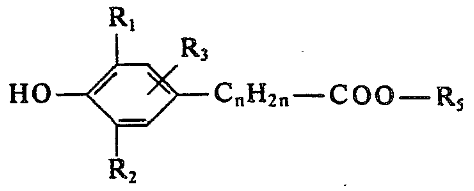

(II)

avec un mercapto-alcool répondant à la formule

$$HO-R_4-SH$$

formules dans lesquelles $R_1$, $R_2$, $R_3$ et $R_4$ ont les significations qui viennent d'être données et $R_5$ représente un alkyle contenant de 1 à 4 atomes de carbone, en présence d'un catalyseur répondant à la formule

$$(R_6O)_4-M$$

dans laquelle les symboles $R_6$ représentant chacun, indépendamment les uns des autres, un radical alkyle contenant de 1 à 18 atomes de carbone, un radical aryle ou un radical alkylaryle et M représente l'un des éléments Ti, Ge, Zr, Sn et V.

2. Procédé selon la revendication 1, pour la préparation de composés répondant à la formule (I)

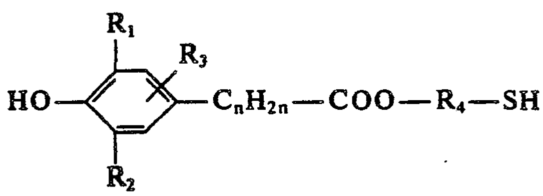

(I)

dans laquelle $R_1$ représente un alkyle en $C_1-C_{18}$, un cycloalkyle, un phényl-1 éthyle ou un phényl-2 isopropyle, $R_2$ représente l'hydrogène ou a la signification de $R_1$, $R_3$ représente l'hydrogène ou un alkyle en $C_1-C_{12}$, $R_4$ représente un alkylène en $C_2-C_{18}$ éventuellement porteur d'un phényle, et un désigne un nombre entier de 0 à 4, procédé caractérisé en ce qu'on transestérifie un composé de formule (II)

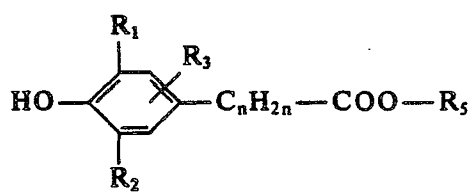

(II)

avec un mercapto-alcool de formule

$$HO-R_4-SH$$

formules dans lesquelles $R_1$, $R_2$, $R_3$, $R_4$ ont les significations qui viennent d'être données et $R_5$ représente un alkyle en $C_1$-$C_4$, en présence d'un catalyseur répondant à la formule

$$(R_6O)_4-M$$

dans laquelle les $R_6$ représentent des radicaux alkyles, identiques ou différents, contenant chacun de 1 à 18 atomes de carbone et M représente l'élément Ti.

3. Procédé selon la revendication 1, caractérisé en ce qu'on utilise, comme catalyseur un ortholitanate de tétra-alkyle dans lequel les alkyles sont identiques ou différents et contiennent chacun de 1 à 4 atomes de carbone.

4. Procédé selon la revendication 1, caractérisé en ce qu'on transestérifie à une température de 50 à 200° C.

5. Procédé selon la revendication 1, caractérisé en ce qu'on transestérifie à une température de 120 à 180° C.

6. Procédé de préparation de composés de formule (I) selon la revendication 1 dans lesquels $R_1$ et $R_2$ représentent chacun un alkyle en $C_1-C_8$, un cyclopentyle ou un cyclohexyle, $R_3$ représente l'hydrogène, $R_4$ représente un alkylène en $C_2-C_4$ ou un phényl-éthylène et n désigne un nombre entier de 1 à 4.

7. Procédé de préparation de composés de formule (I) selon la revendication 1 dans lesquels $R_1$ et $R_2$ représentent chacun un alkyle ramifié en $\alpha$ et contenant de 3 à 8 atomes de carbone, $R_3$ représente l'hydrogène, $R_4$ un radical éthylène, propylène-1,3 ou butylène-1,4 et n désigne le nombre 2 ou le nombre 4.

8. Procédé de préparation du di-tert-butyl-3,5 hydroxy-4 phényl-propionate de mercapto-2 éthyle selon la revendication 1.